# EUROPÄISCHE PATENTSCHRIFT

(11) **EP 0 751 116 B1**
(45) Veröffentlichungstag und Bekanntmachung des Hinweises auf die Patenterteilung: **15.12.1999**
(21) Anmeldenummer: 96106716.2
(22) Anmeldetag: 29.04.1996
(51) Int. Cl.: C07C 221/00, C07C 225/34

(54) **Verfahren zur Herstellung von N',N'-disubstituierten 1,4-Diaminoanthrachinonen**
Process for the preparation of N',N'-disubstituted 1,4-diaminoanthraquinones
Procédé pour la fabrication de 1,4-diaminoanthraquinones N',N'-disubstituées

(30) Priorität: 10.05.1995 DE 19517071
(43) Veröffentlichungstag der Anmeldung: 02.01.1997
(73) Patentinhaber: BAYER AG, 51368 Leverkusen (DE)
(72) Erfinder: Kalz, Dietmar, Dr., 53819 Neunkirchen (DE); Schmitz, Reinold, Dr., 51519 Odenthal (DE); Reinhardt, Karl-Heinz, 51377 Leverkusen (DE); Schröder, Josef, Dr., 51357 Leverkusen (DE); Michaelis, Stephan, Dr., 51519 Odenthal (DE)

(56) Entgegenhaltungen:
- EP-A- 0 095 975
- DE-A- 2 342 469
- US-A- 1 548 768
- US-A- 2 152 191
- US-A- 4 083 683
- PATENT ABSTRACTS OF JAPAN vol. 011, no. 315 (C-451), 14.Oktober 1987 & JP-A-62 101655 (MITSUBISHI CHEM IND LTD), 12.Mai 1987,

## Beschreibung

Die Erfindung betrifft ein verbessertes Verfahren zur Herstellung von N,N'-disubstituierten 1,4-Diaminoanthrachinonen durch Umsetzung von 1,4-Dihydroxyanthrachinonen mit Aminen in Gegenwart von Hydroxycarbonsäuren.

N,N'-disubstituierte 1,4-Diaminoanthrachinone sind beispielsweise als Farbstoffe für Kunststoffe und Synthesefasern als auch als Vorprodukte zur Herstellung von Wollfarbstoffen bekannt. Bislang wurden 1,4-Diaminoanthrachinone hergestellt, indem man 1,4-Dihydroxyanthrachinon (Chinizarin) im Gemisch mit 2,3-Dihydro-1,4-dihydroxyanthrachinon (Leukochinizarin) mit Aminen umsetzt, wobei die Reaktion gegebenenfalls in Gegenwart von Kondensationshilfsmitteln durchgeführt wurde. Als Kondensationshilfsmittel sind beispielsweise Salzsäure (DE-A-2 342 469) oder Essigsäure (US-A-4 083 683) bekannt. Doch selbst bei Verwendung dieser Hilfsmittel sind sowohl die Reaktionszeiten als auch die Ausbeuten nicht optimal.

Es wurde nun ein Verfahren zur Herstellung von N,N'-disubstituierten 1,4-Diaminoanthrachinonen gefunden, das dadurch gekennzeichnet ist, daß man 1,4-Dihydroxyanthrachinone mit aliphatischen oder aromatischen Aminen in Gegenwart von Hydroxycarbonsäuren umsetzt. Die im erfindungsgemäßen Verfahren einzusetzenden Hydroxycarbonsäuren sind vorzugsweise aliphatisch oder aromatisch. In einer besonderen Ausführungsform des erfindungsgemäßen Verfahrens tragen die aliphatischen Hydroxycarbonsäuren die Hydroxy- und Carbonsäuregruppe am selben C-Atom. Die aromatischen Hydroxycarbonsäuren tragen die Hydroxy- und die Carbonsäuregruppe vorzugsweise an zwei unmittelbar benachbarten aromatischen C-Atomen.

Als aliphatische Hydroxycarbonsäuren sind besonders die mit 2 bis 7 C-Atomen bevorzugt. Beispielsweise können genannt werden: Hydroxyessigsäure, Milchsäure, Äpfelsäure, Weinsäure, Zitronensäure, 2,2-Bis-(hydroxymethyl)-propionsäure und Galactonsäure. Besonders bevorzugt ist Hydroxyessigsäure.

Als aromatische Hydroxycarbonsäuren sind insbesondere ortho-Hydroxycarbonsäuren des Benzols oder Naphthalins von Bedeutung. Bevorzugt sind Salicylsäure und ihre Derivate, beispielsweise aliphatische, wie C₁-C₄-Alkyl- oder aromatische, wie C₆-C₁₀-Aryl-Ester, der Formel 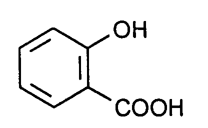 sowie Naphthalin-ortho-hydroxycarbonsäuren und ihre Derivate, beispielsweise aliphatische, wie C₁-C₄-Alkyl- oder aromatische, wie C₆-C₁₀-Aryl-Ester, der Formel 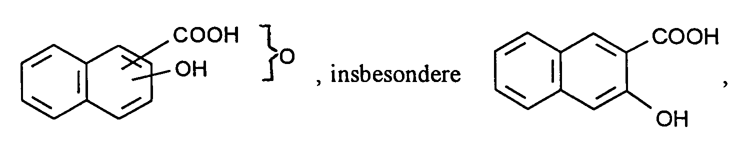 worin O Ortho bedeutet,
die gegebenenfalls durch ein oder mehrere gleiche oder verschiedene Reste R substituiert sind,
wobei
- R: für H, C₁-C₄-Alkyl, insbesondere CH₃, Halogen, insbesondere Cl, Br und F, OH, CN, COOH oder NO₂ steht.

Beispielsweise können genannt werden: 2,5-Dihydroxy-1,4-benzoldicarbonsäure, 2-Naphthol-3-carbonsäure.

Das erfindungsgemäße Verfahren kann dabei in Gegenwart von einer oder mehreren Hydroxycarbonsäuren durchgeführt werden.

Die bevorzugten, bei dem erfindungsgemäßen Verfahren zum Einsatz kommenden aliphatischen oder aromatischen Amine, sind primär. Die aliphatischen Amine können beispielsweise gesättigt, ungesättigt, verzweigt oder geradkettig sein. Besonders bevorzugte aliphatische Amine sind beispielsweise solche der nachstehenden Formeln.

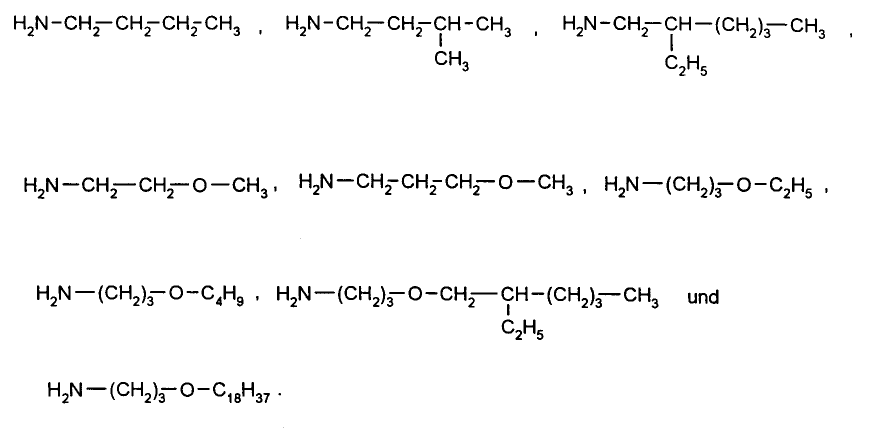

Besonders vorteilhaft wird das erfindungsgemäße Verfahren jedoch zur Herstellung von N,N'-disubstituierten 1,4-Diarylaminoanthrachinonen verwendet, bei denen die aromatischen Amine primär sind, und insbesondere der Formel 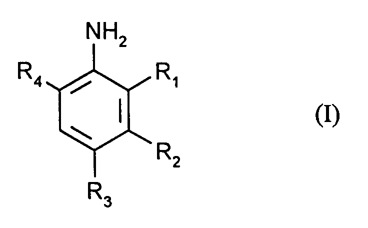 entsprechen, worin
- R₁, R₃ und R₄: unabhängig voneinander für H oder C₁-C₁₂-Alkyl, insbesondere C₁-C₄-Alkyl, bedeuten und
- R₂: für H oder -SO₂-NH-R₅ steht, wobei R₅ für gegebenenfalls substituiertes Aryl, insbesondere C₆-C₁₀-Aryl, wie Phenyl oder Naphthyl, oder Alkyl, insbesondere C₁-C₄-Alkyl, wie Methyl, Ethyl, Propyl oder Butyl, steht und als mögliche Substituenten C₁-C₄-Alkyl, OH, Halogen, C₁-C₄-Alkoxy auch C₉-C₁₀-Aryloxy bevorzugt sind.

Besonders bevorzugt sind aromatische Amine der Formel (I), worin R₁ bis R₄ die nachstehend genannten Bedeutungen haben.

**Tabelle**

| **R**_{**1**} | **R**_{**2**} | **R**_{**3**} | **R**_{**4**} |
|---|---|---|---|
| H | H | CH₃ | H |
| H | H | t.-Bu | H |
| H | H | H | CH₃ |
| CH₃ | H | H | C₂H₅ |
| CH₃ | H | H | CH₃ |
| C₂H₅ | H | H | C₂H₅ |
| CH₃ | H | CH₃ | CH₃ |
| C₂H₅ | H | CH₃ | C₂H₅ |
| CH₃ | SO₂NH-C₆H₅ | CH₃ | CH₃ |

Das im erfindungsgemäßen Verfahren eingesetzte 1,4-Dihydroxyanthrachinon (Chinizarin) wird bevorzugt im Gemisch mit seiner Leukoform dem 2,3-Dihydro-1,4-dihydroxyanthrachinon (Leukochinizarin), vorzugsweise im Verhältnis von 10:90 bis 90:10, insbesondere von 50:50, eingesetzt. Das Gemisch aus Leukochinizarin und Chinizarin kann beispielsweise aus dem Chinizarin durch Zugabe von Reduktionsmitteln wie Zinkstaub oder Natriumdithionit in situ gebildet werden. Die Anthrachinonverbindungen Chinizarin und ihre Leukoform können aber auch separat hergestellt werden. Das Verhältnis von Amin zu Anthrachinonverbindung, d.h. Gesamtmenge aus Chinizarin und Leukochinizarin, wird vorzugsweise so gewählt, daß 1 bis 5 Mol-Äquivalente, insbesondere 2 bis 3,5 Mol-Äquivalente, Amin pro auszutauschende Hydroxygruppe des Anthrachinons (Gesamtmenge an Chinizarin und Leukochinizarin) entfallen.

In einer besonders bevorzugten Ausführungsform wird das erfindungsgemäße Verfahren in Gegenwart von Borsäure durchgeführt. Vorzugsweise wird diese in einer Menge von 0,1 bis 1 Moläquivalent, bezogen auf die auszutauschende Hydroxygruppe des Anthraquinons (Gesamtmenge an Chinizarin und Leukochinizarin), insbesondere 0,25 bis 0,8 Moläquivalente, eingesetzt. Das Verfahren kann gegebenenfalls in Gegenwart von organischen Lösungsmitteln durchgeführt werden. Geeignete Lösungsmittel sind beispielsweise aliphatische Alkohole, wie n-Butanol, iso-Amylalkohol sowie, gegebenenfalls substituierte Aromaten wie Dichlorbenzol, Trichlorbenzol, Toluol, Xylol u.a.. Auch im Überschuß eingesetztes Amin kann als organisches Lösungsmittel dienen.

Das erfindungsgemäße Verfahren wird vorzugsweise bei einer Temperatur von 60 bis 160°C durchgeführt, wobei vorzugsweise gegen Ende der Reaktion eine Temperaturerhöhung vorteilhaft ist, bei der das restliche Reaktionswasser aus dem Reaktionsgemisch entfernt wird.

Die durch eine Hydroxycarbonsäure unterstützte Reaktion läuft deutlich schneller ab als bei Verwendung anderer Kondensationshilfsmittel. Besonders groß ist der Gewinn an Reaktionszeit und Ausbeute bei Verwendung sterisch gehinderter Amine, insbesondere aromatische Amine der Formel (I), worin R₁ und R₄ unabhängig voneinander C₁-C₄-Alkyl bedeuten. Nach Beendigung der erfindungsgemäßen Kondensationsreaktion besitzt die Reaktionsmischung in der Regel eine Temperatur, die vorzugsweise oberhalb der Siedetemperatur von Wasser liegt. Auch Temperaturen darunter sind vorteilhaft, sofern der äußere Druck vermindert worden ist. Die Reaktionsmischung wird nach beendeter Reaktion vorzugsweise abgekühlt. Zur Oxidation von gegebenenfalls vorhandenen Leukoverbindungen wird vorzugsweise Luft durch die Reaktionsmischung geleitet. Die Oxidation kann jedoch auch mit anderen Oxidationsmittel als Sauerstoff durchgeführt werden. Danach folgt in der Regel die Isolierung der 1,4-Diaminoanthrachinonverbindung, die im allgemeinen mit aliphatischen Alkoholen wie Methanol, Ethanol, Propanol, Butanol oder mit Wasser oder Alkoholgemischen gefällt wird. Die 1,4-Diaminoanthrachinonverbindung wird filtriert und vorzugsweise mit den genannten Alkoholen gewaschen. In der Regel schließen sich daran noch Wasserwäschen und schließlich die Trocknung an. Auch die Fällung mit wässriger Salzsäure wie sie aus US-A 4.083.683, Beispiel 1, bekannt ist, kann zur Isolierung verwendet werden.

Die in den Beispielen angegebenen Ausbeuten sind auf die eingesetzte Gesamtmenge an Chinizarin und Leukochinizarin bezogen.

### Beispiel 1 (Vergleichsbeispiel entspricht im wesentlichen Beispiel 1 aus US-A 4.063.683)

In einem Rührkolben mit Destillationsbrücke und Gaseinleitungsrohr wurden
- 280,0 g: (2 Mol) 2-Methyl-6-ethylanilin,
- 40,5 g: (0,166 Mol) Chinizarin,
- 40,5 g: (0,157 Mol) Leukochinizarin,
- 12,8 g: (0,2 Mol) Borsäure und
- 60,7 g: (1,01 Mol) Eisessig
unter Rühren zusammengegeben.

Unter Einleiten von Stickstoff wurde die Temperatur auf 145°C gesteigert. Dabei destillierte ca. 60 g einer Essigsäure/Wasser/Amin-Gemisches ab. Die Temperatur wurde 12 Stunden auf 145°C gehalten, bis die Ausgangsmaterialien verbraucht waren. Dann wurde auf 90°C abgekühlt und während 3 Stunden Luft eingeleitet, nachdem noch 70 g KOH zugesetzt wurden. Anschließend wurde auf 70°C gekühlt und dann 450 ml Methanol zugegeben. Nach Abkühlen auf Raumtemperatur wurde der blaue Farbstoff abfiltriert, erst mit Methanol und danach mit Wasser gewaschen und schließlich im Vakuum getrocknet.
Ausbeute: 107,9 g = 70,5 % der Theorie
Reaktionszeit für die Kondensationsreaktion: 12 Stunden.

### Beispiel 2

In einem Rührkolben mit Destillationsbrücke und Gaseinleitungsrohr wurden
- 280,0 g: (2 Mol) 2-Methyl-6-ethylanilin,
- 40,5 g: (0,166 Mol) Chinizarin,
- 40,5 g: (0,157 Mol) Leukochinizarin,
- 12,8 g: (0,2 Mol) Borsäure und
- 20,0 g: (0,15 Mol) Hydroxyessigsäure, 57 %ig, wäßrig
unter Rühren zusammengegeben.

Unter Einleiten von Stickstoff wurde auf 145°C erwärmt. Dabei destillierten ca. 30 g eines Gemisches aus Wasser und wenig des eingesetzten Amins ab. Nach 5 Stunden waren Chinizarin und Leukochinizarin vollständig umgesetzt. Dann wurde auf 90°C abgekühlt, 50 g KOH zugegeben und 3 Stunden Luft eingeleitet. Danach kühlte man auf 70°C ab und fügte 450 ml Methanol zu. Nach Abkühlen auf Raumtemperatur wurde der blaue Farbstoff abfiltriert, mit Methanol und danach mit Wasser gewaschen und schließlich im Vakuum getrocknet.
Ausbeute: 125,2 g = 81,7 % der Theorie
Reaktionszeit für die Kondensationsreaktion: 5 Stunden.

### Beispiel 3 (Vergleich)

In einem Rührkolben mit Destillationsbrücke und Gaseinleitungsrohr wurden
- 300,0 g: (2,22 Mol) 2,4,6-Trimethylanilin
- 40,5 g: (0,166 Mol) Chinizarin,
- 40,5 g: (0,157 Mol)Leukochinizarin,
- 12,8 g: (0,2 Mol) Borsäure und
- 60,0 g: (1,0 Mol) Eisessig
unter Rühren zusammengegeben.

Unter Einleiten von Stickstoff wurde die Temperatur auf 145°C gesteigert. Dabei destillierten ca. 60 g eines Essigsäure/Wasser/Amin-Gemisches ab. Nach 8 Stunden waren Chinizarin und Leukochinizarin vollständig umgesetzt. Dann wurde auf 85°C abgekühlt. Bei dieser Temperatur wurde nach Zusatz von 50 g KOH 4 Stunden lang Luft eingeleitet. Anschließend wurde auf 70°C abgekühlt und es wurden 450 ml Methanol zugegeben. Nach Abkühlen auf Raumtemperatur wurde der blaue Farbstoff abfiltriert, mit Methanol und mit Wasser gewaschen und schließlich im Vakuum getrocknet.
Ausbeute: 122,5 g = 80 % der Theorie
Reaktionszeit für die Kondensationsreaktion: 8 Stunden.

### Beispiel 4

Ersetzte man in Beispiel 3 die Essigsäure durch 20 g Hydroxyessigsäure, 57 %ig, erhielt man nach einer Kondensationszeit von nur 5 Stunden und weiterer Verfahrensweise wie in Beispiel 3 eine Ausbeute von 131,7 g = 86 % der Theorie.

### Beispiel 5

Ersetzte man in Beispiel 3 die Essigsäure durch 10 g Salicylsäure, erhielt man nach einer Kondensationszeit von 4 Stunden und weiterer Verfahrensweise wie in Beispiel 3 eine Ausbeute von 132 g = 86 % der Theorie.

### Beispiel 6

Ersetzte man in Beispiel 2 die Hydroxyessigsäure durch 14,4 g Milchsäure, 90 %ig, erhielt man nach einer Kondensationszeit von 12 Stunden und weiterer Verfahrensweise wie in Beispiel 2 eine Ausbeute von 127,6 g = 81,7 % der Theorie.
Verwendete man statt Milchsäure 20 g Äpfelsäure, betrug die Kondensationszeit bis zum vollständigen Umsatz 8 Stunden und man erhielt eine Ausbeute von 117 g = 76 % der Theorie.

### Beispiel 7

Ersetzte man in Beispiel 2 die Hydroxyessigsäure durch 18 g 3-Hydroxy-naphthalin-carbonsäure-(2), benötigte man zum vollständigen Umsatz 6 Stunden Kondensationszeit. Bei weiterer Verfahrensweise wie in Beispiel 2 betrug die
Ausbeute: 121 g = 79 % der Theorie.

### Beispiel 8

Ersetzte man in Beispiel 2 die Hydroxyessigsäure durch 23 g 2,6-Dihydroxybenzoesäure, erhielt man nach einer Kondensationszeit von 4 Stunden und weiterer Verfahrensweise wie in Beispiel 2 eine Ausbeute von 120 g = 76,5 % der Theorie. Bei Einsatz von 2,4-Dihydroxy-benzoesäure betrug - bei einer Kondensationszeit von 7 Stunden - die Ausbeute 110,2 g = 72 % der Theorie.

### Beispiel 9

In einem Rührkolben mit Destillationsbrucke und Gaseinleitungsrohr wurden
- 280 g: (1,77 Mol) 2.6-Diethyl-4-methylanilin
- 40.5 g: (0,166 Mol) Chinizarin
- 40,5 g: (0,157 Mol) Leukochinizarin
- 12,8 g: (0,2 Mol) Borsäure und
- 20,0 g: (0,15 Mol) Hydroxyessigsäure, 57%ig, wäßrig
unter Rühren zusammengegeben.

Unter Überleiten von Stickstoff wurde die Temperatur auf 145°C erhöht. Dabei destillierten ca. 30 g eines Gemisches aus Wasser und wenig des eingesetzten Amins ab. Nach 5 Stunden waren Chinizarin und Leukochinizarin fast vollständig verbraucht. Dann wurde auf 90°C abgekühlt, 50 g KOH in Schuppen zugegeben und 3 Stunden lang Luft eingeleitet. Danach kühlte man auf 70°C ab und fügte 450 ml Methanol zu. Nach Abkühlen auf Raumtemperatur wurde der ausgefallene Farbstoff abfiltriert, mit Methanol und dann mit Wasser gewaschen. Schließlich wurde das Produkt im Vakuum getrocknet.
- Ausbeute:: 142 g = 83% der Theorie
- Reaktionszeit:: 5 Stunden.

## Patentansprüche

1. Verfahren zur Herstellung von N,N'-disubstituierten 1,4-Diaminoanthrachinonen, dadurch gekennzeichnet, daß man 1,4-Dihydroxyanthrachinone mit aliphatischen oder aromatischen Aminen in Gegenwart einer Hydroxycarbonsäure umsetzt.

2. Verfahren gemäß Anspruch 1, dadurch gekennzeichnet, daß die Hydroxycarbonsäure aliphatisch oder aromatisch ist.

3. Verfahren gemäß Anspruch 1, dadurch gekennzeichnet, daß die Hydroxycarbonsäure, sofern sie aliphatisch ist, die Hydroxy- und die Carbonsäuregruppe am selben C-Atom und sofern sie aromatisch ist, die Hydroxy- und die Carbonsäuregruppe an zwei unmittelbar benachbarten aromatischen C-Atomen trägt.

4. Verfahren gemäß Anspruch 1, dadurch gekennzeichnet, daß die Hydroxycarbonsäure, sofern sie aliphatisch ist, 2 bis 7 C-Atome besitzt.

5. Verfahren gemäß Anspruch 1, dadurch gekennzeichnet, daß die Hydroxycarbonsäure, sofern sie aromatisch ist, eine ortho-Hydroxycarbonsäure des Benzols oder Naphthalins ist.

6. Verfahren gemäß Anspruch 5, dadurch gekennzeichnet, daß die ortho-Hydroxycarbonsäure der Formel 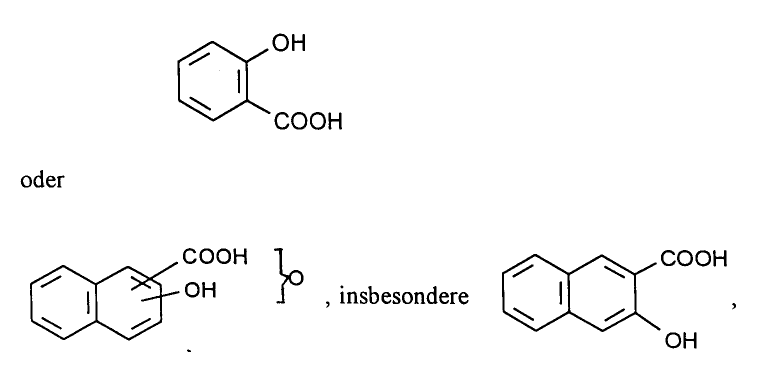 worin O Ortho bedeutet,
entspricht, die gegebenenfalls durch einen oder mehrere gleiche oder verschiedene Reste R substituiert ist, wobei
R für H, C₁-C₄-Alkyl, Halogen, insbesondere Cl, Br und F, OH, CN oder NO₂ steht.

7. Verfahren gemäß Anspruch 1, dadurch gekennzeichnet, daß als Hydroxycarbonsäure Hydroxyessigsäure, Milchsäure, Äpfelsäure, Weinsäure, Zitronensäure, 2,2-Bis-(hydroxymethyl)-propionsäure, Galactonsäure, Salicylsäure, 2,5-Dihydroxy-1,4-benzoldicarbonsäure, und/oder 2-Naphthol-3-carbonsäure eingesetzt wird.

8. Verfahren gemäß Anspruch 1, dadurch gekennzeichnet, daß die aliphatischen oder aromatischen Amine primär sind.

9. Verfahren gemäß Anspruch 1, dadurch gekennzeichnet, daß die aromatischen Amine der Formel 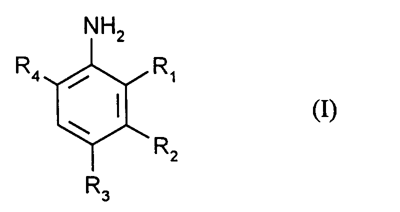 entsprechen, worin
R₁, R₃ und R₄ unabhängig voneinander für H oder C₁-C₁₂-Alkyl, insbesondere C₁-C₄-Alkyl, bedeuten und
R₂ für Wasserstoff oder -SO₂-NH-R₅ steht, wobei R₅ für gegebenenfalls substituiertes Aryl oder Alkyl steht.

10. Verfahren gemäß Anspruch 1, dadurch gekennzeichnet, daß man in Gegenwart von Borsäure arbeitet.

## Claims

1. Process for the preparation of N,N'-disubstituted 1,4-diaminoanthraquinones, characterized in that 1,4-dihydroxyanthraquinones are reacted with aliphatic or aromatic amines in the presence of a hydroxycarboxylic acid.

2. Process according to Claim 1, characterized in that the hydroxycarboxylic acid is aliphatic or aromatic.

3. Process according to Claim 1, characterized in that the hydroxycarboxylic acid if aliphatic carries the hydroxyl and the carboxylic acid group on the same carbon atom and if aromatic carries the hydroxyl and the carboxylic acid group on two directly adjacent aromatic carbon atoms.

4. Process according to Claim 1, characterized in that the hydroxycarboxylic acid if aliphatic has 2 to 7 carbon atoms.

5. Process according to Claim 1, characterized in that the hydroxycarboxylic acid if aromatic is an ortho-hydroxycarboxylic acid of benzene or naphthalene.

6. Process according to Claim 5, characterized in that the ortho-hydroxycarboxylic acid is of the formula 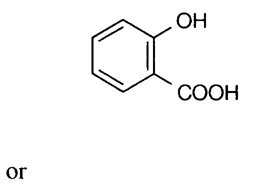 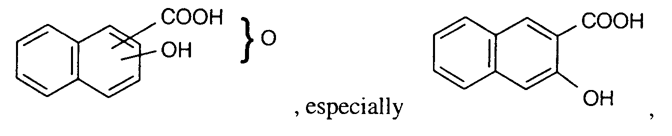 in which O is ortho,
which is optionally substituted by one or more identical or different radicals R, in which
R represents H, C₁-C₄-alkyl, halogen, especially Cl, Br and F, OH, CN or NO₂.

7. Process according to Claim 1, characterized in that the hydroxycarboxylic acid employed is hydroxyacetic acid, lactic acid, malic acid, tartaric acid, citric acid, 2,2-bis-(hydroxymethyl)-propionic acid, galactonic acid, salicylic acid, 2,5-dihydroxy-1,4-benzenedicarboxylic acid, and/or 2-naphthol-3-carboxylic acid.

8. Process according to Claim 1, characterized in that the aliphatic or aromatic amines are primary.

9. Process according to Claim 1, characterized in that the aromatic amines are of the formula 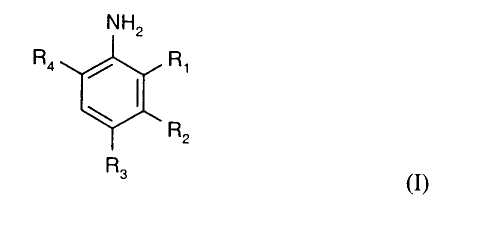 in which
R₁, R₃ and R₄ independently of one another represent H or C₁-C₁₂-alkyl, especially C₁-C₄-alkyl, and
R₂ represents hydrogen or -SO₂-NH-R₅, in which R₅ represents optionally substituted aryl or alkyl.

10. Process according to Claim 1, characterized in that it is carried out in the presence of boric acid.

## Revendications

1. Procédé pour la préparation de 1,4-diaminoanthraquinones N,N'-disubstituées, caractérisé en ce que l'on fait réagir des 1,4-dihydroxyanthraquinones avec des amines aliphatiques ou aromatiques en présence d'un acide hydroxycarboxylique.

2. Procédé selon la revendication 1, caractérisé en ce que l'acide hydroxycarboxylique est aliphatique ou aromatique.

3. Procédé selon la revendication 1, caractérisé en ce que l'acide hydroxycarboxylique, lorsqu'il est aliphatique, porte le groupe hydroxy et le groupe acide carboxylique sur le même atome de carbone, et lorsqu'il est aromatique, il porte le groupe hydroxy et le groupe acide carboxylique sur deux atomes de carbone aromatiques immédiatement voisins.

4. Procédé selon la revendication 1, caractérisé en ce que l'acide hydroxycarboxylique, lorsqu'il est aliphatique, contient 2 à 7 atomes de carbone.

5. Procédé selon la revendication 1, caractérisé en ce que l'acide hydroxycarboxylique, lorsqu'il est aromatique, consiste en un acide orthohydroxycarboxylique dérivé du benzène ou du naphtalène.

6. Procédé selon la revendication 5, caractérisé en ce que l'acide orthohydroxycarboxylique répond à la formule 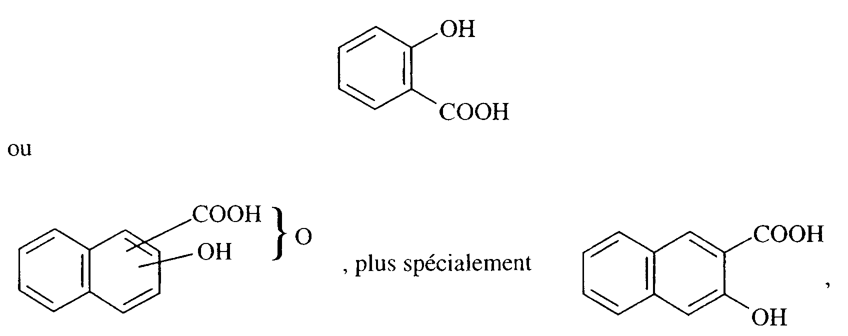 dans laquelle O indique la position ortho,
avec le cas échéant un ou plusieurs substituants R identiques ou différents,
R représentant H, un groupe alkyle en C₁-C₄, un halogène, plus spécialement Cl, Br ou F, un groupe OH, CN, ou NO₂.

7. Procédé selon la revendication 1, caractérisé en ce que l'acide hydroxycarboxylique utilisé est l'acide hydroxyacétique, l'acide lactique, l'acide malique, l'acide tartrique, l'acide citrique, l'acide 2,2-bis-(hydroxyméthyl)-propionique, l'acide galactonique, l'acide salicylique, l'acide 2,5-dihydroxy-1,4-benzènedicarboxylique et/ou l'acide 2-naphtol-3-carboxylique.

8. Procédé selon la revendication 1, caractérisé en ce que les amines aliphatiques ou aromatiques sont primaires.

9. Procédé selon la revendication 1, caractérisé en ce que les amines aromatiques répondent à la formule 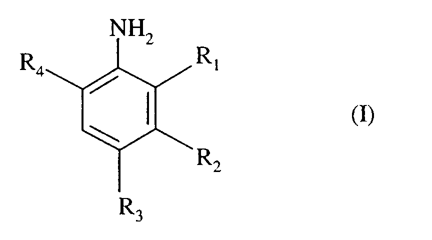 dans laquelle
R₁, R₃ et R₄ représentent chacun, indépendamment les uns des autres, H ou un groupe alkyle en C₁-C₁₂, plus spécialement alkyle en C₁-C₄ et
R₂ représente l'hydrogène ou un groupe -SO₂-NH-R₅ dans lequel R₅ représente un groupe aryle ou alkyle éventuellement substitué.

10. Procédé selon la revendication 1, caractérisé en ce l'on opère en présence d'acide borique.
